(19) Europäisches Patentamt
European Patent Office
Office européen des brevets

(11) Publication number: 0 119 933 B1

(12) EUROPEAN PATENT SPECIFICATION

(45) Date of publication of patent specification: 04.03.87

(51) Int. Cl.4: B 01 J 23/72, C 07 C 19/045, C 07 C 17/156

(21) Application number: 84400556.1

(22) Date of filing: 20.03.84

# (54) Supported catalyst for the synthesis of 1-2 dichloroethane by oxychlorination of ethylene within a fluidized bed.

(30) Priority: 22.03.83 IT 2019883

(43) Date of publication of application:
26.09.84 Bulletin 84/39

(45) Publication of the grant of the patent:
04.03.87 Bulletin 87/10

(84) Designated Contracting States:
BE DE FR GB IT NL

(56) References cited:
EP-A-0 057 796

The file contains technical information submitted after the application was filed and not included in this specification

(73) Proprietor: AUSIMONT S.p.A.
31, Foro Buonaparte
I-20121 Milano (IT)

(72) Inventor: Cavaterra, Enrico
21 Via 4 Novembre
Saronno (Varese) (IT)
Inventor: Bossi, Alessandro
10 Via Mantegna
I-28100 Novara (IT)

(74) Representative: Hirsch, Marc-Roger
Cabinet Hirsch 34 rue de Bassano
F-75008 Paris (FR)

EP 0 119 933 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

The invention concerns a supported particulate catalyst for the synthesis of 1,2-dichloroethane (by means of $C_2H_4$ oxychlorination within a fluidized bed) comprising a Cu compound in amounts corresponding to a Cu content from 1 to 10% by weight—as Cu metal—obtained by dry impregnation (with the solution of a Cu compound) of a carrier preferably selected from the group consisting of microspheroidal $Al_2O_3$, microspheroidal $SiO_2$ and microspheroidal silica-alumina. The invention also concerns a method for the preparation of the said catalyst. In the present description and in the claims which follow, the sum of the moles of Al and Si (and of any other possible metal contained in the carrier) is indicated as Σ [Me] and the moles of copper, present in the catalyst as a whole, are indicated by [Cu].

The catalytic gaseous phase oxychlorination of $C_2H_4$ with HCl and $O_2$ (or gases containing same) is well known and is widely used for the preparation of DCE, from which, in a subsequent stage (by pyrolysis), vinyl chloride is obtained.

Likewise it is known to use the catalytic mass in the form of a fluidized bed, in order to get rid of the considerable heat released by both the oxychlorination reaction and the combustion of ethylene to CO and $CO_2$; the fluidized bed processes which realize conditions of almost a perfect isothermicity are those which up to now have achieved the best results.

Catalytic compositions promoting the oxychlorination have already been described. The most common one is based on copper compounds, in general chlorides, supported in different amounts of microspheroidal supports (in general $Al_2O_3$) suitable, due to their granulometric distribution and resistance to friction wear, for use in fluidized beds. The previously described compositions present, however, many drawbacks which limit, and in certain instances hinder, satisfactory exploitation on an industrial scale. Thus, for instance, there are catalytic formulations characterized by high DCE yields with respect to HCl, which must work, however, with a high $C_2H_4$ excess, with respect to HCl, in order to prevent the phenomena of bad fluidization (even defluidization), whereby the DCE yields, with respect to ethylene, are rather unsatisfactory. The $C_2H_4$ excess, with respect to DCE, cannot, in fact, be converted into DCE and is therefore eliminated with the gaseous exhausts as unreacted $C_2H_4$ or in the form of carbon oxides (CO and $CO_2$).

On the other hand there are compositions which can work with a lower excess of $C_2H_4$, with respect to HCl, without incurring particular problems of bad fluidization of the catalytic bed, thus operating with high DCE yields with respect to $C_2H_4$, but which, on the contrary, lead to low HCl conversions. In the latter case it is necessary to use special equipment, withstanding HCl, in order to avoid corrosion. Furthermore it is necessary to neutralize the unconverted HCl with alkali, with a consequential increase in technical requirements. Moreover, certain compositions are characterized by the use of carriers different from $Al_2O_3$, for instance $SiO_2$ or silica alumina, or by the presence in the catalyst, together with $CuCl_2$, of other compounds, in general chlorides, of alkali or alkaline-earth metals and of rare earths.

The following documents may be cited:

— Italian Patent 690,193, teaching the use of compositions based on $CuCl_2$, alkaline-earth chlorides and rare earth chlorides, in order to reduce the extent of the combustion on a fluidized bed;
— British Patent 971,996, which exemplifies catalytic compositions based on $CuCl_2$ and alkaline-earth chlorides on either a fixed or fluidized bed.

None of these compositions overcomes completely all the drawbacks, with respect to a satisfactory industrial exploitation, namely:

— insufficient DCE yield, with respect to $C_2H_4$;
— insufficient conversion of HCl;
— poor fluidization (even defluidization).

In order to better appreciate the importance of these drawbacks and, consequently, the importance of overcoming them, it may be useful to provide further specific information as to the bad fluidization phenomena, typical of the catalysts described herein-above. The reaction may be represented as follows:

$$C_2H_4+2HCl+1/2\ O_2=Cl—CH_2CH_2—Cl+H_2O$$

which means that in order to obtain a 100% DCE yield, with respect to $C_2H_4$, a mixture of HCl and $C_2H_4$ in a molar ratio of at least 2 must be fed besides oxygen. Thus, for instance, if HCl and $C_2H_4$, in a 1.86 molar ratio, is fed into the reactor, the maximum theoretical DCE yield, with respect to $C_2H_4$ is: 1.86:2×100=93%.

The yields that can be obtained are usually below this value, because of incomplete conversion of HCl; in fact, if:

R=$HCl/C_2H_4$ molar feed ratio;

$$C=\%\text{ of conversion of HCl}=\left(1-\frac{\text{HCl moles at outlet}}{\text{HCl moles at inlet}}\right)\times 100,$$

are used, it can be shown that the DCE yield, with respect to ethylene, is expressed by the equation: DCE yield=(R/2)×C, so that, in order to obtain high yields, R and C must be both as high as possible. Within a fluidized bed reactor, consisting of a catalyst based on $CuCl_2$, supported on microspheroidal $Al_2O_3$, prepared according to known technologies, the following phenomena occur:

— with relatively low $HCl/C_2H_4$ feed ratios, (e.g. lower than 1.9 moles) the fluidization is good, but, for the reasons stated hereinabove, the DCE yield, with respect to ethylene, is limited (e.g. below 95%).
— increasing the $HCl/C_2H_4$ ratio, above the previously indicated values, worsens the fluidization and decreases the HCl conversion. This worsening can be perceived visually in pilot glass reactors and reveals itself by the formation of an increasing number of gas bubbles of increasing diameter. When the diameter of the bubbles equals roughly the diameter of the reactor, the "rupture" of the catalytic bed can occur, i.e. inside the bed the formation of "empty" zones, alternated by "full" zones, can be seen. Under extreme conditions, the catalyst is dragged out of the reactor.

In industrial reactors the worsening reveals itself in a less striking but still evident way through the undue loss of catalyst in the cyclones, due to the clogging of the cyclones' legs, which hinders the flowing back, into the catalyst bed, of the catalyst separated at the head of the cyclones. In both cases it is impossible to carry out normal operations and it is necessary to decrease the $HCl/C_2H_4$ feed ratio until a good fluidization is restored or, in extreme cases, to switch off the feed of the reactants.

The causes of these phenomena have been generically ascribed to the so called "stickiness", that hinders the free moving or reciprocal creep of the single granules, within the catalytic bed, because of the formation of clots that are difficult to fluidize (whereby the bubbles) and slightly flowable (whereby the clogging of cyclones' legs).

The sticking can be observed only in connection with the increase of the $HCl/C_2H_4$ feed ratio and is reversible. These indications mean that the active part of the catalyst namely Cu is responsible for said phenomena (rather than the carrier, whose features do not substantially depend on said ratio). In fact, if the results of many kinetic works are analysed, it is possible to trace nearly always a mechanism involving:

1. the reaction of $C_2H_4$ with $Cu_2Cl_4$, in order to give DCE and $Cu_2Cl_2$;
2. the oxidation of $CuCl_2$ with $O_2$ (air), to give $Cu_2OCl_2$ and $H_2O$;
3. the reaction of 2HCl with $Cu_2OCl_2$ to give again $Cu_2Cl_4$ and $H_2O$.

In other words Cu shifts cyclically from $Cu_2Cl_4$ (chlorided Cu) to $Cu_2Cl_2$ (reduced Cu) to $Cu_2OCl_2$ (oxychlorided Cu) and at last again to $Cu_2Cl_4$. The equilibrium between such forms depends essentially on the $HCl/C_2H_4$ feed ratio; when the ratio increases the chlorided form becomes prevailing, while at low values of said ratio the oxychlorided form is prevailing.

The occurrence of sticking may thus be explained by a predominance of $Cu_2Cl_4$ on increase of the $HCl/C_2H_4$ ratio. On the other hand, there is evidence of the trend of $Cu_2Cl_4$ to form polymers (Kenney C. N., Catal. Rev. Sci. Eng. 11 (2), 197 (1975)):

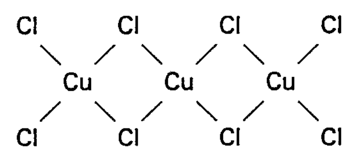

The invention also concerns a method for the preparation of the said catalyst. The article published in Ind. Eng. Chem. Prod. Res. Dev., vol 20 No. 3, 1981 page 435 (3) "Eggyolk distribution" and page 441, right-hand column) describes the production of a catalyst particle with a higher concentration of catalytic metal at the centre than at the surface by using a competitive agent (HCl, $MgCl_2$) during impregnation. This document, however, does not teach a method for the preparation of a catalyst according to the present invention and can only constitute a technical background to the invention.

One object of the present invention is that of providing in the most simple and effective way, a catalyst for the $C_2H_4$ oxychlorination to DCE, suitable for use in fluidized beds, displaying best the properties required in view of a satisfactory industrial exploitation, namely:

— a high DCE yield, with respect to $C_2H_4$;
— a high conversion of HCl;
— excellent fluidization properties.

The invention concerns a supported catalyst for the synthesis of 1,2-dichloroethane characterized in that the molar ratio

$$\frac{\text{outer Me}}{\text{outer Cu}}$$

(as determined by X-Ray Photoemission Spectroscopy using the K-alpha radiation of Mg and integrating the photoemission peaks, to give an intensity value which, after correction by a respective sensitivity factor, is directly proportioned to the surface atomic concentration of the respective elements) is at least 40% higher than the molar ratio:

$$y=\frac{\Sigma\ [Me]}{[Cu]}$$

According to a preferred aspect of the invention the total amount of Cu present in the said catalyst as a whole is comprised between 3 and 8% by weight.

Preferably the molar ratio (outer Me/outer Cu) is equal to or higher than 20:1 and the Cu concentration on the outer surface of the carrier is zero or at least much lower than the Cu concentration in the layers immediately underlying the analyzed surface layer. Interesting results were obtained with a ratio (outer Me/outer Cu) between 40:1 and 53:1. Said catalyst comprises advantageously also a Mg compound, the Mg amounts being usually from 0.2 to 0.8 moles of Mg per mole of Cu.

In order to obtain a catalyst characterized by low Cu concentration on the outer surface of the granules and having, as such, the features required by a satisfactory industrial exploitation, different ways may be used and particularly the following two methods.

I) The first method is characterized in that a preformed catalyst of the usual type, based on $CuCl_2$ supported e.g. on $Al_2O_3$, is impregnated in its dry state with an aqueous solution (of a volume substantially equal to the volume of the pores of the carrier), containing a strong acid, for instance HCl, in amounts corresponding to at least 1 equivalent per mole of Cu (present in the catalyst) and preferably also containing $MgCl_2$ in amounts from 0 to 1 mole per mole of Cu (present in the catalyst), the catalyst being preferably dried (e.g. at 110°C) after impregnation, and subsequently activated in air (e.g. for 4 hours between 180°C and 300°C). In this way it is possible to obtain a catalyst from the outer surface of which the major portion of Cu (present in the starting pre-formed catalyst) was removed.

II) The second method is characterized in that a carrier is impregnated in the dry state with an aqueous solution (of a volume substantially equal to the volume of the pores of the carrier) containing a Cu compound, in such an amount as to load into the final catalyst, a Cu amount, expressed as metal, from 1 to 10% by weight, and containing, moreover, an acid and a Mg compound, in amounts equal to at least 1 equivalent of acid and from 0 to 1 mole of Mg per mole of Cu, the catalyst being preferably dried, after impregnation, and then being activated in air between 180° and 300°C.

Also in this case it is possible to obtain a catalyst on whose outer surface the Cu content is kept at a minimum. The Mg compound corresponds preferably to the Cu compound in the sense that, for instance, in the case where salts are used, the Mg salt is derived from the same acid as the Cu salt. Analogously said salt must correspond to the Cu compound. If the Cu compound is $CuCl_2$, the acid will thus be HCl while the Mg compound will be $MgCl_2$.

The catalyst according to the invention is characterized by a minimum or zero (and at any rate definitely lower than that of the catalysts already described) concentration of Cu on the outer surface of the granule, as shown by results from the determinations carried out with the technique known as X-Ray Photoemission Spectroscopy (henceforth XPS), and, as such, displays excellent fluidization properties and maintains high HCl conversions, even in the case of high $HCl/C_2H_4$ ratios so that it is possible to reach high DCE yields with respect to $C_2H_4$. The inner Cu, although acting in the conversion cycles and in the equilibria between the various forms ($CuCl_2$, $Cu_2Cl_4$, etc.), depending on the $HCl/C_2H_4$ feed ratio, does not give rise to the sticking phenomena which, on the contrary, involve zones of possible contact between the different granules.

The main feature of the catalyst according to the invention, is that the active part is almost completely segregated inside the pores of the carrier, whereby the Cu concentration on the outer surface is at a minimum, and any way lower than that of conventional catalysts, as shown by XPS measurements, and at said minimum the concentration also remains after long periods of time.

The use of this catalyst allows the reaction to be performed within a fluidized bed, using, without any bad fluidization, high $HCl/C_2H_4$ ratios in the feed and (particularly when the catalyst contains Mg) high conversions of HCl and high DCE yields (with respect to $C_2H_4$) are thus obtained, in accordance with the equation: yield of $DCE=R\times C/2$.

The operative oxychlorination conditions do not substantially differ from those typical for the catalysts previously described.

$C_2H_4$, HCl and gases containing $O_2$ (in general air), fed in gaseous phase, are then pre-heated to a temperature from 200 to 250°C, preferably from 220° to 235°C.

The other operative parameters, in general, are comprised between the following ranges:

A) $Air/C_2H_4$ ratio: such that the $O_2$ content, in the gaseous exhausts, after condensation of DCE, $H_2O$ and HCl, is from 3 to 10% by volume.

B) $HCl/C_2H_4$ ratio: the nearest possible to the stoichiometric value (2/1 molar), compatible with good

fluidization conditions of the catalytic bed and of a sufficiently high conversion of HCl, conditions which depend on the type of catalyst.

C) Contact time (expressed as a ratio between the volume of the catalytic bed in a fluidized state, and the volumetric flow rate of the mixture of reactants, at the temperature and pressure conditions existing in the catalytic bed): it depends essentially on the type of the catalyst used; in general it is from 10 to 40, preferably from 20 to 30 seconds.

D) Linear velocity of the gases: within the range between the minimum fluidization rate and the dragging speed, both being typical for each type of catalyst; in general said velocity is from 10 to 50, preferably from 20 to 40 cm/s.

E) Total pressure during the reaction (relevant for achieving an effective contact between the reactants, in a gaseous phase, and the catalyst, in a solid phase): in general pressures greater than atmospheric pressure and up to 600 kPa are used; at greater pressures, energy waste becomes predominant, due to the compression work.

The following examples are given for purely non-limitative illustrative purposes.

Operative conditions common to different examples

The catalysts cited in the examples (except preformed catalyst 'A') were prepared by impregnation in the dry state with an aqueous solution, dried at 110°C and activated in air for 4 hours at the temperature (either 180° or 300°C) indicated on Table 1, which also reports the results of the XPS analysis. The measurements of the outer surface concentration of Cu were carried out following the XPS technique (cf. C. D. Wagner: "Handbook of X-ray Photoemission Spectroscopy" Perkin Elmer Co., Eden Prairie; 1979), based on X-ray irradiation and on measuring the energy level and the energy intensity of the electrons emitted by the solid. The energy level of said electrons is characteristic for the element while the energy intensity is proportional to the number of atoms present in the volume of sample, to a depth substantially from 2 to 3 nm (20—30 Å) from the surface. As the mean granulometric size of the catalyst is around 50 µm (micrometers) and usually ranges from 20 to 80 µm are cited, the measures of the atomic concentrations refer to about 1 tenthousandth of the diameter of the granule, namely, essentially, to its outer surface. A small amount of sample (just a few mg) was pressed onto a pure indium plate in order to obtain an analyzable surface of an area equivalent to a few sq.mm. The samples were then analyzed under a high-pushed vacuum, at a basic pressure of $2\times10^{-7}$ Pa, using an X-ray source operating at 400 W and fitted with a Mg-anode (K alpha radiation of magnesium). The photoemission spectra of the present elements, that is 0/1s, C/1s, Cl/2p, Mg/2p, Al/2p, were then gathered together under conditions of high resolution by using a computer suitable for the digitalized acquisition of the data, with a maximation of the signal noise ratio. After removal of the background noise, the areas of the photoemission peaks were calculated by means of numerical integration. The intensity value thus obtained, corrected for the respective sensitivity factor, was directly proportional to the surface atomical concentration of the respective element.

Examples from 1 to 6 (preparation of the catalyst)

Catalyst 'A' is a usual (commercially available) preformed comparison catalyst, based on $CuCl_2$, supported on a microspheroidal alumina carrier, having a Cu content (as a metal) of 5% by weight, while comparison catalyst 'B' was prepared by impregnation in the dry state of a carrier (consisting of microspheroidal alumina with a mean particle diameter of 50 µm) with an aqueous solution (with a volume equal to the volume of the pores of the carrier) containing $CuCl_2$, thereby obtaining a catalyst with a final Cu content (expressed as a metal) of 5% by weight. The outer coating degree can be better represented by the Al/Cu ratio which, independently from possible variations in the corresponding amounts of oxygen and chlorine, gives the "frequency" of Cu atoms with respect to Al atoms. In this way the outer surface of catalysts A and B is characterized by the presence of one Cu atom every 10 Al atoms, as indicated on Table 1.

Catalyst 'A/1' was prepared by impregnating in the dry state catalyst 'A' with an aqueous solution (of a volume equal to the volume of the pores of the catalyst), and containing 2 moles of HCl and 0,75 mole of $MgCl_2$ per moles of Cu (contained in the catalyst). According to Table 1, the outer surface Cu is reduced fourfold (4 times) with respect to catalyst A.

Catalyst 'B/1' was prepared by impregnating in the dry state the carrier used for preparing catalyst B with an aqueous solution (of a volume equal to the volume of the pores of the carrier). Said solution contained:

— $CuCl_2$ in such an amount to obtain 5% by weight of Cu on the global weight of the catalyst;
— 2 moles of HCl per mole of Cu;
— 0,75 moles of $MgCl_2$ per mole of Cu.

According to Table 1, catalyst B/1 having an outer surface Cu concentration reduced four to fivefold with respect to catalyst B. Let us now summarize hereinbelow the most significant ratios.

| Ex. | Catalyst | $X=\frac{\text{outer Al}}{\text{outer Cu}}$ (by moles) | $Y=\frac{\text{total Al}}{\text{total Cu}}$ (by moles) | $K=\frac{X-Y}{Y}\times 100$ |
|---|---|---|---|---|
| 1* | A* | 10 | 22.3 | negative (−55.16%) |
| 2* | B* | 10 | 22.3 | negative (−55.16%) |
| 3 | A/1 | 40 | 22.3 | 44.25% |
| 4 | A/1 | 40 | 22.3 | 44.25% |
| 5 | B/1 | 45 | 22.3 | 50.44% |
| 6 | B/1 | 53 | 22.3 | 57.92% |

* Comparative.

TABLE 1

| Ex. No. | Catalyst | Activation temperature T (°C) | % of atoms on external surface | | | | Al/Cu |
|---|---|---|---|---|---|---|---|
| | | | Cu | Cl | Al | O | |
| 1 | A* | — | 3.0 | 4.4 | 32.4 | 60.2 | 10 |
| 2 | B* | 180 | 3.8 | 4.9 | 38.8 | 52.4 | 10 |
| 3 | A/1 | 180 | 0.9 | 3.8 | 36.0 | 59.3 | 40 |
| 4 | A/1 | 300 | 1.0 | 4.0 | 40.0 | 55.0 | 40 |
| 5 | B/1 | 180 | 0.8 | 4.6 | 35.4 | 59.2 | 45 |
| 6 | B/1 | 300 | 0.7 | 4.2 | 37.0 | 58.1 | 53 |

* Comparison samples

TABLE 2

| Ex. No. | Catalyst | Al/Cu | Activation temperature T (°C) | Oxychlorination T (°C) | $HCl/C_2H_4$ (by moles) | DCE yield (molar % on fed $C_2H_4$) | HCl conversion (%) | Fluidization |
|---|---|---|---|---|---|---|---|---|
| 7 | A | 10 | — | 220 | 1.871 | 93.45 | 99.89 | good |
| 8 | A | 10 | — | 220 | 1.913 | 95.35 | 99.69 | good |
| 9 | A | 10 | — | 220 | 1.936 | 96.35 | 99.53 | bad |
| 10 | A | 10 | — | 220 | 1.958 | 97.00 | 99.08 | defluidizes |
| 11 | A/1 | 40 | 180 | 225 | 1.844 | 91.90 | 99.89 | excellent |
| 12 | A/1 | 40 | 180 | 225 | 1.936 | 96.25 | 99.40 | excellent |
| 13 | A/1 | 40 | 180 | 225 | 2.014 | 97.80 | 97.12 | good |
| 14 | A/1 | 40 | 300 | 225 | 1.889 | 94.20 | 99.70 | excellent |
| 15 | A/1 | 40 | 300 | 225 | 1.945 | 96.55 | 99.28 | excellent |
| 16 | A/1 | 40 | 300 | 225 | 2.016 | 97.70 | 96.93 | good |
| 17 | B | 10 | 180 | 220 | 1.920 | 95.47 | 99.54 | good |
| 18 | B | 10 | 180 | 220 | 1.980 | 97.05 | 98.10 | bad |
| 19 | B | 10 | 180 | 220 | 2.029 | — | — | defluidizes |
| 20 | B/1 | 45 | 180 | 225 | 1.840 | 91.90 | 99.83 | excellent |
| 21 | B/1 | 45 | 180 | 225 | 1.889 | 94.20 | 99.75 | excellent |
| 22 | B/1 | 45 | 180 | 225 | 1.945 | 96.59 | 99.30 | excellent |
| 23 | B/1 | 45 | 180 | 225 | 2.016 | 97.68 | 96.90 | good |
| 24 | B/1 | 53 | 300 | 225 | 1.936 | 96.24 | 99.40 | excellent |
| 25 | B/1 | 53 | 300 | 225 | 2.014 | 97.86 | 97.20 | good |
| 26 | B/1 | 53 | 300 | 225 | 2.089 | 97.93 | 93.80 | good |

Examples from 7 to 26 (behaviour of the catalyst)

Into a glass reactor having a diameter of 4 cm and a height of 3 m., suitable for withstanding pressures up to 600 kPa, were introduced the prepared catalysts and then they were tested in the form of a fluidized bed (in the oxychlorination of $C_2H_4$) under the following conditions:

. P=4 absolute atm.;

. contact time: 28 sec.;

. air/$C_2H_4$=3.2 (by moles).

The values of oxychlorination temperature and of the ratio R=HCl/$C_2H_4$, are reported on Table 2, along with the results.

Examples 7 to 16 show that, corresponding to the lower concentrations of outer surface Cu, the better fluidization properties of catalysts A/1 allow to operate at higher HCl/$C_2H_4$ ratios, so as to provide better DCE yields with respect to catalyst A. The comparison becomes even more evident from the graph in figure 1. The same may be said for examples from 17 to 26 and therefore for the excellence of catalysts B/1 with respect to catalyst B (see fig. 2).

**Claims**

1. A supported particulate catalyst for the synthesis of 1,2-dichloroethane (by means of $C_2H_4$ oxychlorination within a fluidized bed) comprising a Cu compound in amounts corresponding to a Cu content from 1 to 10% by weight—as Cu metal—obtained by dry impregnation (with a solution of a Cu compound) of a carrier preferably selected from the group consisting of microspheroidal $Al_2O_3$, microspheroidal $SiO_2$ and microspheroidal silica-alumina, wherein the sum of the moles of Al and Si (and of any other possible metal contained in the carrier) is indicated hereinafter as Σ [Me] and wherein the moles of copper, present in the catalyst as a whole, are indicated hereinafter by [Cu], characterized by the fact that the molar ratio:

$$\frac{\text{outer Me}}{\text{outer Cu}}$$

(as determined by X-Ray Photoemission Spectroscopy using the K-alpha radiation of Mg and integrating the photoemission peaks, to give an intensity value which, after correction by a respective sensitivity factor, is directly proportioned to the surface atomic concentration of the respective elements) is at least 40% higher than the molar ratio:

$$y=\frac{\Sigma\,[\text{Me}]}{[\text{Cu}]}$$

2. A catalyst according to claim 1, wherein the total amount of Cu present in the catalyst as a whole, is from 3 to 8% by weight.

3. A catalyst according to claim 1, wherein the molar ratio (outer Me/outer Cu) is equal to or higher than 20:1.

4. A catalyst according to claim 1, wherein said catalyst also comprises a Mg compound, the Mg amount being from 0 to 1 and particularly from 0.2 to 0.8 moles per mole of Cu.

5. A method for the preparation of a catalyst according to any of claims 1 to 4, characterized in that a preformed catalyst, based on $CuCl_2$ supported on $Al_2O_3$, $SiO_2$ or silica-alumina, is loaded, by means of a dry impregnation, with an aqueous solution (of a volume substantially equal to the volume of the carrier's pores) containing a strong acid, e.g. HCl, in amounts corresponding to at least 1 equivalent per mole of Cu (present in the catalyst), said acid solution also preferably containing a Mg compound in an amount of up to 1 moles of Mg per mole of Cu (present in the catalyst), whereafter the catalyst is activated in the air at 180—300°C.

6. A method for the preparation of a catalyst according to claims 1 to 4, characterized in that a carrier is loaded, by means of a dry impregnation, with an aqueous solution (of a volume substantially equal to the volume of the carrier's pores) containing a Cu compound in such amounts so as to produce, in the final catalyst, a Cu content (expressed as a metal) from 1 to 10% by weight, and containing, moreover, an acid and a Mg compound, in amounts equal to at least 1 equivalent of acid and between 0 and 1 mole of Mg per moles of Cu respectively, whereafter the catalyst is activated in the air at 180—300°C.

7. A method according to claim 6, wherein said Mg compound corresponds to the Cu compound in the sense that, in the case where salts are used, the Mg salt is derived from the same acid as the Cu salt.

8. A method according to claim 7, wherein said acid corresponds to the Cu compound.

9. A method according to claim 7 or 8, wherein the Cu compound is $CuCl_2$, the acid is HCl and the Mg compound is $MgCl_2$.

**Patentansprüche**

1. Getragener, feinzerteilter Katalysator zur Synthese von 1,2-Dichloräthan (durch Oxychlorierung von $C_2H_4$ in einer Wirbelschicht), mit einer vorliegenden Cu-Verbindung in einer einem Cu-Gehalt von 1—10 Gew.-%—an metallischem Kupfer—entsprechenden Menge, hergestellt durch Trockenimpregnierung (mittels einer Lösung einer vorliegenden Cu-Verbindung) eines Trägers, der vorzugsweise aus mikrosphäroidem $Al_2O_3$, aus mikrosphäroidem $SiO_2$ oder aus mikrosphäroider Kieseltonerde besteht, wobei die Summe der Al-Mole und Si-Mole (und der Mole jeglicher underer ggf. im Träger vorliegenden Metall-Mole) nachstehend durch $\Sigma$ {Me} und die Anzahl der im gesamten Katalysator vorliegenden Kupfer-Mole nachstehend durch {Cu} dargestellt ist, dadurch gekennzeichnet, dass das Molarverhältnis

$$\frac{\text{äusseres Me}}{\text{äusseres Cu}}$$

(bestimmt durch Lichtstrahlungs-Roentgenspektroskopische Untersuchung vermittels der K-alpha-Strahlung von Mg, mit Integrierung der Lichtstrahlungsspitze, zwecks Ermittlung eines Intensitätswertes der nach Berichtigung vermittels eines entsprechenden Empfindlichkeitsfaktors zur Oberflächen-Atomkonzentration der betreffenden Elemente direkt proportional ist), um wenigstens 40% grösser ist, als das Molarverhältnis:

$$y=\frac{\Sigma\ \{Me\}}{\{Cu\}}$$

2. Katalysator nach Anspruch 1, bei dem die Gesamtmenge des im gesamten Katalysator vorliegenden Cu insgesamt 3 bis 8 Ge.-% beträgt.

3. Katalysator nach Anspruch 1, bei dem das Molarverhältnis "ausseres Me/äusseres Cu" gleich oder grösser als 20/1 ist.

4. Katalysator nach Anspruch 1, bei dem der Katalysator ferner eine Mg-Verbindung enthält, derart, dass die Menge an Mg 0 bis 1, insbesondere 0,2 bis 0,8 Mol pro Mol C beträgt.

5. Verfahren zur Herstellung eines Katalysators nach einem der Ansprüche 1 bis 4, dadurch gekennzeichnet, dass man einen vorgefertigten, von $Al_2O_3$, $SiO_2$ oder Kieseltonerde getragenen Katalysator auf $CuCl_2$-Basis durch Trockenimpregnierung mit einer wässerigen Lösung (mit einem dem Volumen der Trägerporen wesentlichen gleichen Volumen) chargiert, die eine starke Säure, wie HCl enthält, und zwar in Mengen, die wenigstens einem Äquivalent pro Mol Cu (das im Katalysator vorliegt), wobei die saure Lösung vorzugsweise ebenfalls eine Mg-Verbindung enthält, in einer Menge bis zu 1 Mol Mg pro Mol Cu (das im Katalysator vorliegt), wonach man den Katalysator unter Luftzutritt bei 180—300°C aktiviert.

6. Verfahren zur Herstellung eines Katalysators nach den Ansprüchen 1 bis 4, dadurch gekennzeichnet, dass man den Träger durch Trockenimpregnierung mit einer wässerigen Lösung (mit einem dem Volumen der Trägerporen wesentlich gleichen Volumen) chargiert, die eine Cu-Verbindung in einer derartigen Menge enthält, dass der fertige Katalysator einen Cu-Gehalt (ausgedrückt in metallischem Kupfer) von 1 bis 10 Gew.-% besitzt, wobei die Lösung ferner eine Säure und eine Mg-Verbindung enthält in Mengen, die wenigstens einem Äquivalent Säure bzw. 0 bis 1 Mol Mg pro Mol Cu gleich sind, wonach man den Katalysator unter Luftzutritt bei 180 bis 300°C aktiviert.

7. Verfahren nach Anspruch 6, bei dem die Mg-Verbindung insofern der Cu-Verbindung entspricht, als bei Verwendung von Salzen das Mg-Salz von der gleichen Säure hergeleitet ist, wie das Cu-Salz.

8. Verfahren nach Anspruch 7, bei dem die Säure der Cu-Verbindung entspricht.

9. Verfahren nach Anspruch 7 oder 8, bei dem die Cu-Verbindung $CuCl_2$ ist, wobei die Säure HCl ist und die Mg-Verbindung $MgCl_2$ ist.

**Revendications**

1. Catalyseur particulaire supporté, pour la synthèse de 1,2-dichloroéthane (par oxychloration de $C_2H_4$ dans un lit fluidisé), renfermant un composé de Cu en proportion correspondant à une teneur en Cu de 1 à 10% en poids—en Cu métallique—et obtenu par imprégnation à sec (utilisant une solution d'un composé de Cu) d'un support choisi, de préférence, parmi les suivants: $Al_2O_3$ microsphéroïde, $SiO_2$ microsphéroïde et silice-alumine microsphéroïde, dans lequel la somme de moles de Al et Si (et de tout autre métal éventuellement présent dans le support) la désigne ci-dessus par $\Sigma$ {Me}, et dans lequel les moles de cuivre présent dans la totalité du catalyseur sont indiquées par {Cu}, caractérisé par le fait que le rapport molaire:

$$\frac{\text{Me externe}}{\text{Cu externe}}$$

(tel que d terminé par spectroscopie à photoémission aux rayons-X, avec utilisation du rayonnement K-alpha de Mg et intégration des crêtes de photoémission en vue de l'obtention d'une valeur d'intensité qui, après correction par un facteur de sensibilité respectif, est directement proportionnelle à la concentration atomique de surface des éléments respectifs) est supérieur d'au moins 40% au rapport molaire:

$$y = \frac{\Sigma \{Me\}}{\{Cu\}}$$

2. Catalyseur selon la revendication 1, dans lequel la quantité totale de Cu présent dans la totalité du catalyseur est de 3 à 8% en poids.

3. Catalyseur selon la revendication 1, dans lequel le rapport molaire "Me externe/Cu externe" est égal ou supérieur à 20/1.

4. Catalyseur selon la revendication 1, dans lequel ledit catalyseur renferme également un composé de Mg, la quantité de Mg étant de 0 à 1, plus particulièrement de 0,2 à 0,8 mole par mole de Cu.

5. Procédé de préparation d'un catalyseur selon une des revendications 1 à 4, caractérisé en ce qu'un catalyseur préformé à base de $CuCl_2$, supporté sur de $Al_2O_3$, $SiO_2$ ou de la silice-alumine est chargé par imprégnation sèche d'une solution aqueuse (en volume sensiblement égal à celui des pores du support) contenant un acide fort tel que HCl en quantité correspondant à au moins 1 équivalent par mole de Cu (présent dans le catalyseur, ladite solution acide renfermant également de préférence un composé de Mg en quantité égale, au plus, de 1 mole par mole de Cu (présente dans le catalyseur), après quoi le catalyseur est activé à l'air à 180—300°C.

6. Procédé de préparation d'un catalyseur selon les revendications 1 à 4, caractérisé en ce qu'on charge un support, par imprégnation sèche, d'une solution aqueuse (en volume sensiblement égal à celui des pores du support) renfermant un composé de Cu en quantité telle qu'il en résulte, dans le catalyseur fini, une teneur en Cu (exprimé en Cu métallique) de 1 à 10% en poids, en renfermant, en outre, un acide et un composé de Mg en quantités égales à au moins 1 équivalent d'acide et de 0 à 1 mole de Mg par mole de cuivre, respectivement, après quoi l'on active le catalyseur à l'air à 180—300°C.

7. Procédé selon la revendication 6, dans lequel ledit composé de Mg correspond au composé de Cu en sens que, lorsqu'on utilise des sels, le sel de Mg est dérivé du même acide que le sel de Cu.

8. Procédé selon la revendication 7, dans lequel ledit acide correspond au composé du Cu.

9. Procédé selon la revendication 7 ou 8, dans lequel le composé de cuivre est de $CuCl_2$, l'acide est le HCl et le composé de Mg est le $MgCl_2$.

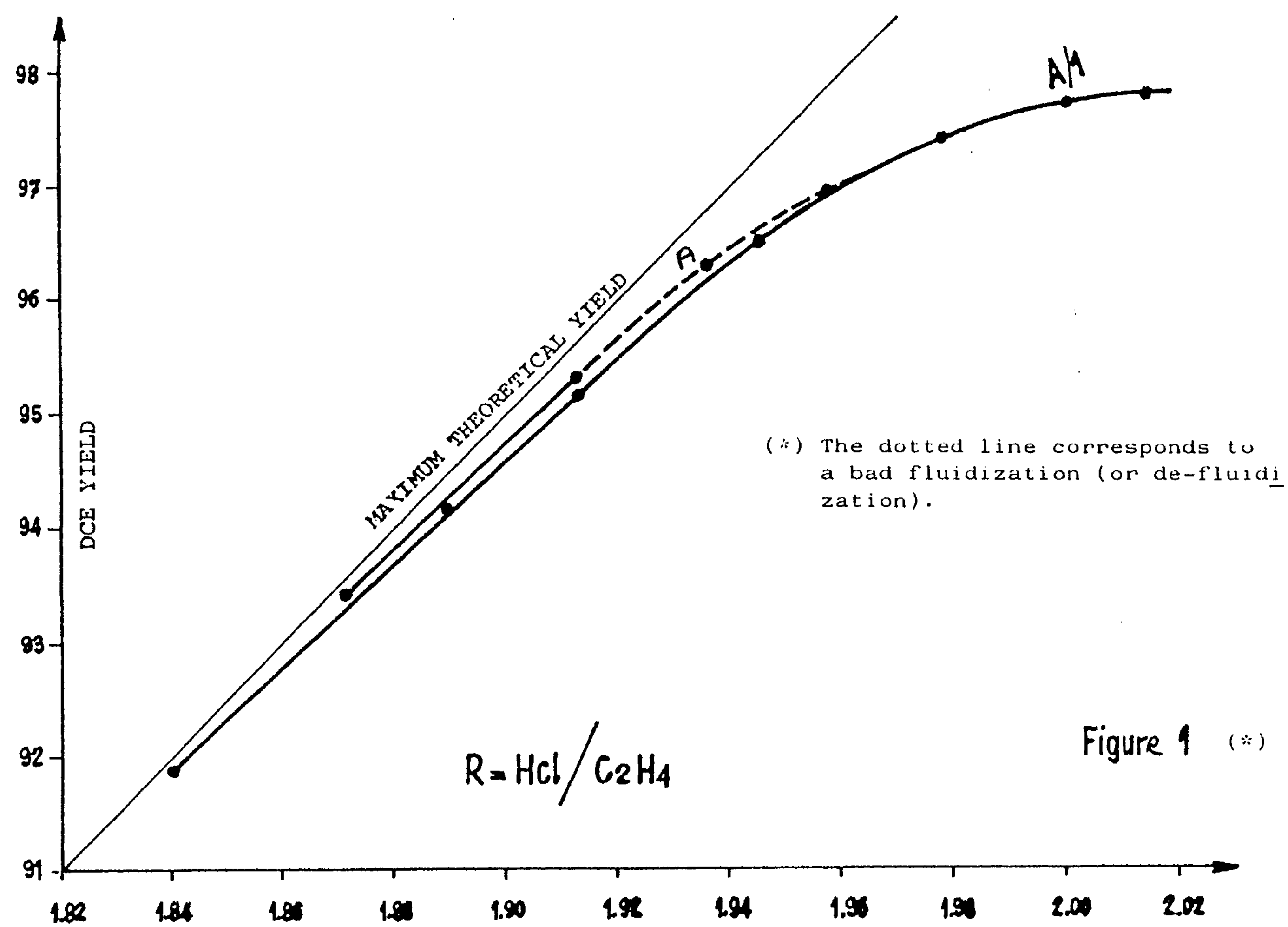
DCE YIELD
MAXIMUM THEORETICAL YIELD
A
A/A
R = HCl/C2H4
98
97
96
95
94
93
92
91
1.82
1.84
1.86
1.88
1.90
1.92
1.94
1.96
1.98
2.00
2.02
(*) The dotted line corresponds to a bad fluidization (or de-fluidization).

Figure 1 (*)

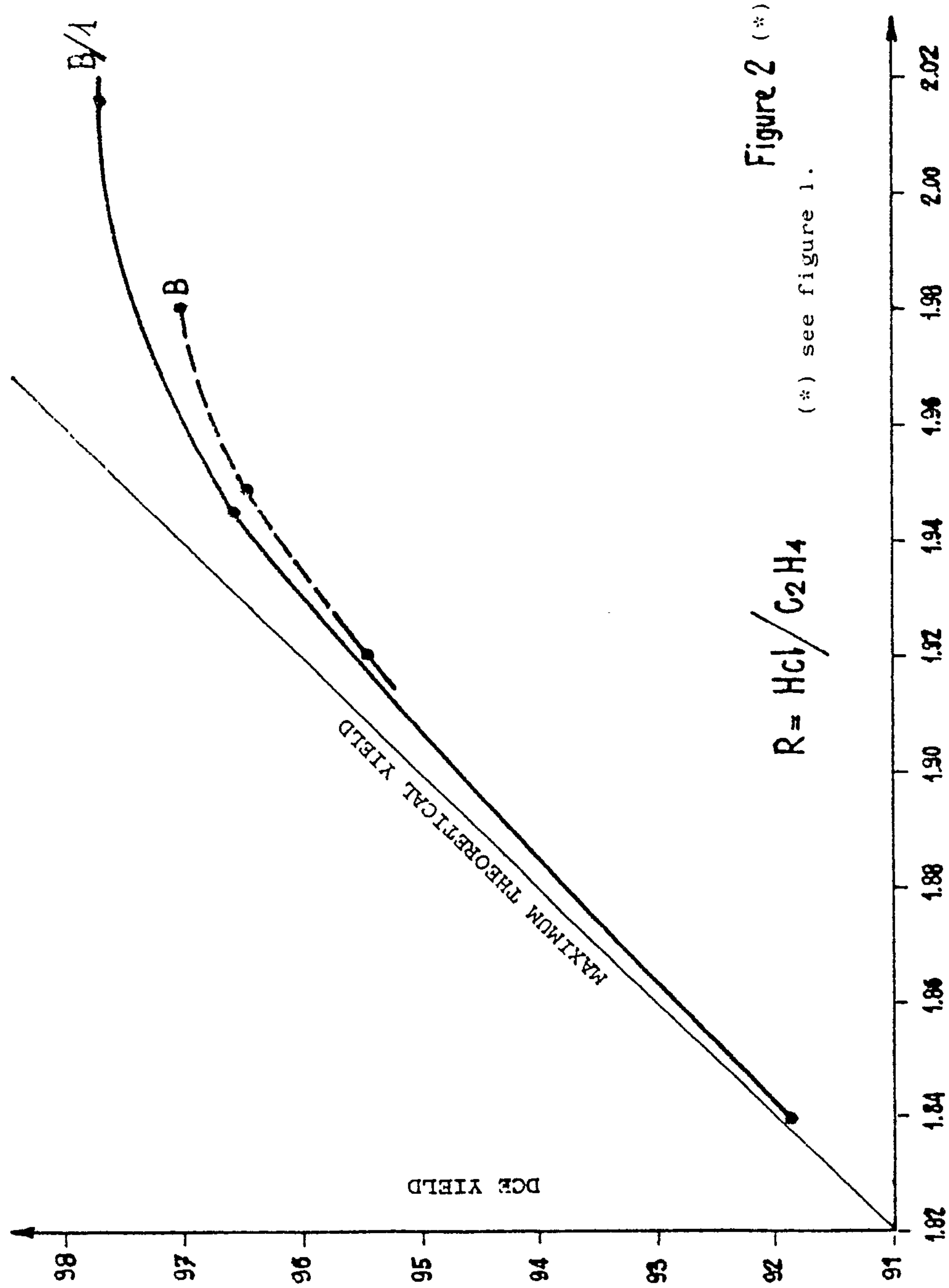


Figure 2 (*)

(*) see figure 1.